Europäisches Patentamt

European Patent Office

Office européen des brevets

(11) Publication number: **0 263 403 B1**

# EUROPEAN PATENT SPECIFICATION

(45) Date of publication of patent specification: **21.04.93**

(51) Int. Cl.⁵: **C12N 1/20**, C12P 17/00, C12P 7/00, //(C12N1/20, C12R1:01)

(21) Application number: **87114172.7**

(22) Date of filing: **29.09.87**

(54) **Novel microorganism and method for producing oxygenous compounds with said microorganism.**

(30) Priority: **03.10.86 JP 234415/86**

(43) Date of publication of application:
**13.04.88 Bulletin 88/15**

(45) Publication of the grant of the patent:
**21.04.93 Bulletin 93/16**

(84) Designated Contracting States:
**BE CH DE FR GB IT LI NL SE**

(56) References cited:
EP-A- 0 088 602
GB-A- 1 603 864
GB-A- 2 018 822
GB-A- 2 019 390

JOURNAL OF GENERAL MICROBIOLOGY, vol. 61, 1970, Colchester(GB); R.WHITTENBURY et al., pp. 205-218&NUM;

DETERMINATIVE BACTERIOLOGY, 8th ed., 1975, The Williams & Wilkins Co., Baltimore, MD (US); R.E. BUCHMANN et al., p. 269&NUM;

(73) Proprietor: **IDEMITSU KOSAN COMPANY LIMITED**
**1-1, Marunouchi 3-chome Chiyoda-ku Tokyo(JP)**

(72) Inventor: **Uchiumi, Toshiki, c/o Idemitsu Kosan Co.Ltd.**
**No. 1280, Kamiizumi, Sodegaura-machi Kimitsu-gun, Chiba-ken(JP)**
Inventor: **Muramoto, Takahisa, c/o Idemitsu Kosan Co.Ltd.**
**No. 1280, Kamiizumi, Sodegaura-machi Kimitsu-gun, Chiba-ken(JP)**
Inventor: **Suzuki, Motoshi, c/o Idemitsu Kosan Co.Ltd.**
**No. 1280, Kamiizumi, Sodegaura-machi Kimitsu-gun, Chiba-ken(JP)**

(74) Representative: **Türk, Dietmar, Dr. rer. nat. et al**
**Türk, Gille, Hrabal, Leifert Patentanwälte Brucknerstrasse 20**
**W-4000 Düsseldorf 13 (DE)**

**Description**

BACKGROUND OF THE INVENTION

The present invention relates to a novel microorganism, particularly a novel microorganism belonging to a genus Mythylococcus, and a method for producing oxygenous compounds with said microorganism.

Methane-utilizing bacteria can be grown using methane and methanol as the sole carbon and energy sources, which is stably supplied at low prices and in large amounts, and the obtained microorganisms (biomasses) can be utilized as the protein sources.

Further, it is known to oxidize alkanes, alkenes, alicyclic compounds, aromatic compounds and heterocyclic compounds with methane monooxygenase retained in the methane-utilizing bacteria, thereby obtaining the corresponding compounds (Japanese Patent Publication No. 58-31198, Japanese Patent Laid-Open Publication No. 57-65187 and Japanese Patent Laid-Open Publication No. 58-47496).

With these methods intended for practical use, however, some problems remain unsolved in connection with contamination due to infectious microbes, stability of enzymes and recovery of the products.

Use of methane-utilizing bacteria capable of growing at elevated temperatures and having stable enzymatic activity have been considered to be effective for the solution of such problems. Not until now are such methane-utilizing bacteria found. See GB-A-2 018 822.

SUMMARY OF THE INVENTION

As a result of screening for microorganisms which can be grown at elevated temperatures, while using methane and methanol as the sole carbon and energy sources, it has been found that obligately methane-utilizing bacteria was isolated from a soil in Okinawa Prefecture, Japan, can grow at a temperature of up to 53.5 ° C, and retain stable methane monooxygenase.

According to one aspect of the present invention, there is provided a novel microorganism Methylococcus sp. SIM-222 (FERM BP-1470) which belongs to a genus Methylococcus, and can be grown, using peptone or casamino acid as the nitrogen source.

According to another aspect of the present invention, there is provided a method for producing oxygenous compounds characterized in that said microorganism is allowed to come into catalytic contact with an oxidizable organic compound containing at least two carbon atoms under aerobic conditions.

DETAILED DESCRIPTION OF THE INVENTION

The present inventors have isolated the desired microorganisms from a soil in Okinawa Prefecture, Japan, by the following procedures.

Firstly, the culture medium components specified in Table 1 were dissolved in 1 ℓ of distilled water, and the obtained solution was adjusted to pH 7 to prepare a culture medium.

Table 1

| Na$_2$HPO$_4$·12H$_2$O | 1.5 g |
|---|---|
| KH$_2$PO$_4$ | 0.5 g |
| MgSO$_4$·7H$_2$O | 0.2 g |
| CaCl$_2$·2H$_2$O | 0.05 g |
| MnSO$_4$·4H$_2$O | 5 mg |
| FeSO$_4$·7H$_2$O | 5 mg |
| CoSO$_4$·7H$_2$O | 5 mg |
| CuSO$_4$·5H$_2$O | 5 mg |
| Nicotinic acid | 200 $\mu$g |
| Biotin | 2 $\mu$g |
| Calcium pantothenate | 400 $\mu$g |
| Folic acid | 2 $\mu$g |
| Inositol | 1000 $\mu$g |
| p-aminobenzoate | 200 $\mu$g |
| Pyridoxine·HCl | 400 $\mu$g |
| Riboflavin | 200 $\mu$g |
| Thiamine·HCl | 400 $\mu$g |

The medium (50 ml) was poured in a flask of 700 ml in volume, and was sterilized under pressure at 120°C for 15 minutes. After cooling, 1 g of a soil sample was put in the flask, and the gaseous phase was replaced with a gas mixture consisting of 40% by volume of methane, 50% by volume of air and 10% by volume of CO$_2$. Thereafter, cultivation was carried out at 52°C for 7 days.

An inoculating loop of cells from the obtained culture solution was streaked on a plate medium prepared by adding 20 g of agar to 1 ℓ of the same medium as used in the aforesaid incubation. This was incubated at 52°C for 7 days in a mixed gas atmosphere consisting of 40% by volume of methane, 50% by volume of air and 10% by volume of CO$_2$. The resultant colony was isolated to obtain the desired microorganism.

The microorganism was then classified according to the classification and identification method of bacteria (Bergey's Manual of Systematic Bacteriology, the first edition, 1984). In what follows, the results of the identification testing of the microorganism will be tabulated along with those of the known bacteria.

Table 2

| Particulars | Present Bacteria | Methylococcus | Methylomonas | Methylocinus | Methylobacterium |
|---|---|---|---|---|---|
| Structure of Intracellular Membranes* | Type I | Type I | Type I | Type II | Type II |
| Major Carbon Assimilation Pathway | Ribulose Monophosphate Cycle | Ribulose Monophosphate Cycle | Ribulose Monophosphate Cycle | Serine Cycle | Serine Cycle |
| TCA Cycle | Imcomplete | Imcomplete | Imcomplete | Complete | Complete |
| Nitrogenase | + | d** | d** | + | + |
| Predominant Fatty Acid Carbon Chain Length | 16 | 16 | 16 | 18 | 18 |
| Growth on Glucose | – | – | – | – | + |
| NAD or NADP Dependency of Isocitric Acid Dehydrogenase | NAD | NAD | NAD NADP | NADP | NADP |
| Cell Shape | Cocci | Cocci | Cocci or Rods | Rods or Vibrios | Rods |
| Growth at 45°C | + | + | d** | – | – |

*According to R. Whittenbury et al., J. C. M. (1970), 61, 205-218.

**11-89% of the strains are positive

From the foregoing results, this strain is recognized as belonging to a genus Methylcoccus. Further, this strain has the following mycological features.

```
Growth at 26°C                                      +

Growth at 37°C                                      +

Growth at 45°C                                      +

Growth at 53.5°C                                    +

Motility                                            -

Formation of Capsule                                +

Color of Colony                   Red Brown

Gram's Staining                                     -

Catalase                                            +

Oxidase                                             +

Growth at pH                      5.5 - 8.5

Concentration resistant to Methanol          50 g/ℓ

Concentration resistant to Propylene Oxide 10 mmol/ℓ

Concentration resistant to Butyrene Oxide  12 mmol/ℓ

Concentration resistant to Copper Sulfate 100 mg/ℓ

Compounds Usable as Carbon Source:  Methane, Methanol

Compounds Usable as Nitrogen Source:  Nitrate such as
      potassium nitrate; Ammonium Salts such as ammonium
      chloride; Amino Acid, Casamino Acid, Peptone.
```

Still further, this strain can be grown, using peptone and casamino acid as the sole nitrogen source in the presence of methane, and show a growth rate higher than that obtained when a nitrate salts is supplied as the sole nitrogen source. Not until now is any report made on the strains belonging to the genus Methylococcus and having such mycological features.

The microorganism of this invention catalyze the following reactions.

Octane → Octanol
Propene → 1,2-Epoxypropane
1-Butene → 1,2-Epoxybutane
Benzene → Phenol

It has hitherto be known that the methane monooxygenase in methane-utilizing bacteria oxidize various types of substrates. Hence, the microorganism of this invention is also presumed to be capable to oxidize the substrates, as is the case with other methane-utilizing bacteria.

From the foregoing mycological properties, the present inventors have judged the above strain to be novel bacteria belonging to the genus Methylococcus, and termed the microorganism of this invention Methylococcus sp. SIM-222 strain. This strain has already been deposited with Fermentation Research Institute, Agency of Industrial Science and Technology under No. FERM P-8830 (FERM BP-1470).

The microorganism of this invention is capable of coming into catalytic contact with oxidizable organic compounds containing at least two carbon atoms under aerobic conditions, thereby producing the corresponding oxygenous compounds.

In the culture conditions for producing said oxygenous compounds, a culture medium having the composition as specified in Table 3 is used as the basic medium, to which the required carbon and energy sources are optionally added.

<u>Table 3</u>

| | |
|---|---|
| $KNO_3$ | 1 g |
| $NH_4Cl$ | 0.1 g |
| $(NH_4)_2SO_4$ | 0.1 g |
| $MgSO_4 \cdot 7H_2O$ | 2.0 g |
| $CaCl_2 \cdot 2H_2O$ | 0.2 g |
| $KH_2PO_4$ | 0.52 g |
| $NaHPO_4 \cdot 12H_2O$ | 1.43 g |
| EDTA·Ferric ammonium salt | 8 mg |
| Tris(hydroxymethyl)aminomethane | 2.4 g |
| 3-(N-morpholino)propane sulfonic acid | 10.5 g |
| $Na_2MoO_4 \cdot 2H_2O$ | 1 mg |
| Trace metal solution* | 2 mℓ |
| Distilled water | 1 ℓ |

adjusted to pH 6.83 with potassium hydroxide

*Composition of Trace Metal Solution

| | |
|---|---|
| $CuSO_4 \cdot 5H_2O$ | 1700 mg |
| $FeSO_4 \cdot 7H_2O$ | 500 mg |
| $ZnSO_4 \cdot 7H_2O$ | 400 mg |
| $H_3BO_3$ | 15 mg |
| $CoCl_2 \cdot 6H_2O$ | 50 mg |
| EDTA·disodium salt | 250 mg |
| $MnCl_2 \cdot 4H_2O$ | 20 mg |
| $NiCl_2 \cdot 6H_2O$ | 10 mg |
| Distilled water | 1 ℓ |

It may be used methane and/or methanol as the carbon source. The nitrogen source applied may include amino acid, casamino acid and peptone in addition to the compounds specified in Table 3. As the energy source (electron donor), formaldehyde, formic acid or its salt, ethanol, hydrogen, NADH and so on may optionally be added besides the aforesaid methane and methanol.

Examples of the oxidizable organic compounds containing at least two carbon atoms to be used as the substrates include alkanes such as propane, butane, pentane and octane; alkenes such as propene, butene, pentene and octene; ethers such as dimethyl ether, diethyl ether and methyl ethyl ether; alicyclic compounds such as cyclopentane, cyclohexane, cyclooctane, cyclodecane, cyclopentene, cyclohexene, cyclooctene and cyclodecene; aromatic compounds such as benzene, toluene, xylene, biphenyl, indene, naphthalene, tetralin and anthracene; and heterocyclic compounds such as thiophene, pyridine, thiazole, pyrimidine, triazine and quinoline.

The methane-utilizing bacteria, which oxidize such substrates, are strict aerobic bacteria, and may be cultured either batchwisely or continuously under aerobic conditions at 25 to 55°C, preferably 26 to 53.5°C, more preferably 30 to 50°C and pH 5.5 to 8.5, preferably 6.5 to 7.5.

The cultured biomasses may be directly used as the enzyme source. However, it is preferred to use the microbial biomasses obtained by solid-liquid separation achieved as by centrifuging. Further, the microbial biomasses may be washed with a suitable solution such as a phosphate buffer solution, and be suspended in said solution for use. Still further, the microbial biomasses may be used in the form of crushed biomasses, purified enzymes, immobilized biomasses, immobilized enzymes and so on. The crushed biomasses may be obtained by crushing a suspension of microbial biomasses by means of ultrasonic waves, French press or high-pressure homogenizers, and may then be subjected to solid-liquid separation and purified into purified enzymes by suitable purification means. Immobilization of the microbial biomasses or enzymes may also be achieved in the conventional manner. Use of the immobilized biomasses or enzymes assures efficient reactions. It is to be understood that the oxidization reaction may be carried out at a temperature of 25 to 55°C and pH of 5.5 to 8.5. It is preferred that to the reaction, the aforesaid electron donor is optionally added.

The oxygenous compounds corresponding to the substrates, such as alcohols, oxides, phenols and quinones are obtained by this oxidation reaction.

The present invention provides novel methane-utilizing bacteria capable of growing at elevated temperatures, and makes it possible to use such microorganisms or their treated masses as the enzyme sources for the oxidation reaction of various organic compounds. In this case, such enzyme sources can be used over an extended period of time, since their activity can be recovered. Further, even when methanol is used as the carbon source, the methane-utilizing bacteria of this invention has the property of high methane monooxigenase activity. Still further, the methane-utilizing bacteria of this invention is much higher in the resistance to oxides such as propylene oxide and butylene oxide than the known bacterium.

The present invention can effectively be applied to a variety of fields inclusive of foods and chemical industries.

In the following, Examples are given to illustrate the present invention in more detail.

Example 1

(1) Separation of Microorganism

The medium components specified in Table 1 were dissolved in 1 ℓ of distilled water, and was adjusted to pH 7.0. Ten (10) ml of this medium were put in a test tube having a volume of 60 ml, and were sterilized under pressure at 120°C for 15 minutes. After cooling, 1 g of a mud sample collected in the vicinity of Yonagusuku, Nakagami-Gun, Okinawa Prefecture, Japan, was put in the test tube, and the gaseous phase was replaced by a gas mixture consisting of 40% by volume of methane, 50% by volume of air and 10% by volume of $CO_2$. With a reciprocating shaker, incubation was carried out at 52°C for 7 days. A culture medium having the same composition was inoculated with 0.5 ml of the obtained culture solution, followed by incubation under the same conditions. One inoculating loop of this culture solution was streaked on a plate medium prepared by adding 20 g of agar to 1 ℓ of the same medium. Incubation was carried out at 52°C for 7 days in a mixed gas atmosphere having the same composition as mentioned above.

The thus obtained colony was isolated, and was again streaked on the same plate medium for incubation at 52°C in the same atmosphere. The obtained colony was re-isolated, and was inoculated on 10 ml of a liquid medium having the same composition. Afterwards, while the gaseous phase was replaced by a gas mixture of the same composition as mentioned above, the colony was grown to separate strain (Methylococcus sp. SIM-222, FERM BP-1470).

(2) Incubation using methane and methanol as the carbon and energy sources

The medium components specified in Table 3 were dissolved in 1 ℓ of distilled water, and the resulting solution was adjusted to pH 6.83. Fifty (50) ml of this medium were put in a vessel of 700 ml in volume, and were sterilized under pressure at 120°C for 15 minutes. After cooling, 60 ml of methane and 0.25 ml of methanol were added to the sealed vessel. It was inoculated with 2.5 ml of the bacterial culture solution obtained in the item (1) for incubation at 45°C for 24 hours with a reciprocating shaker (120 reciprocations/min.). Turbidimetry indicated that the degree of turbidity of the culture solution after 24 hours was 2.8, as expressed in terms of the absorbance of light having a wavelength of 540 nm.

(3) Incubation using methane as the carbon source

Incubation was carried out according to the procedures of the item (2), except that the gaseous phase was replaced by a gaseous mixture of methane : air = 1 : 1, and methane was used as the sole carbon source. After 24 hours, the culture solution showed an absorbance of 1.0 at 540 nm.

(4) Incubation using methanol as the carbon source

Five hundred (500) ml of a culture medium of the same composition as used in the item (2) were put in a vessel of 1000 ml in volume, and were sterilized under pressure at 120°C for 15 minutes. After cooling, 50 ml of the obtained culture solution which was incubated in the same manner as used in the item (2) were inoculated to the vessel, and methanol was added as the carbon source in such a manner that its concentration was adjusted to 0.2%. Thereafter, the temperature was maintained at 45°C, and stirring was under an aeration of 50 ml/min. at 300 rpm. The medium was regulated to pH 6.8 to 6.9 by automatic addition of 1 N nitric acid and 1 N sodium hydroxide with a pH controller. As the biomasses increased, the amount of dissolved oxygen decreased and reached zero, but started to increase again. Simultaneously with increases in the amount of dissolved oxygen, methanol was added in such a manner that its concentration was 0.2%. Twenty-four (24) hours after the initiation of incubation, methanol was continuously supplied. Methanol was dissolved in a culture medium of the same composition as used for incubation in such a manner that its concentration amounted to 60%, and was supplied at a rate of 2 ml/hr. An aeration of 1000 ml/min. and stirring of 700 rpm were applied. Forty-eight (48) hours after the initiation of incubation, 10 ml of a concentrated liquid (10 times) having the same composition as used for incubation was filtrated through a filter having a pore diameter (0.45 μm), and was then added to the culture solution. After the lapse of 68 hours from the initiation of incubation, 8 g/ℓ of the dry biomasses in the culture solution were obtained.

Example 2

Fifty (50) ml of the bacterial culture solution obtained in the items (2) of Example 1 were centrifuged at 5000 x g for 10 minutes, and were further centrifugally washed twice under the same conditions. The washing liquid used was a liquid containing the medium components except for potassium dihydrogen phosphate, disodium hydrogen phosphate, potassium nitrate, ammonium sulfate and ammonium chloride. After washing, the biomasses (Methylococcus sp. SIM-222, FERM BP-1470) were resuspended in the same solution, and the bacterial concentration of the suspension was adjusted in such a manner that the absorbance was 3 at 540 nm. The activity of methane monooxygenase of this bacterial suspension was measured.

Five (5) ml of propylene were added to two test tubes sealed with caps, each of 10 ml in volume, by means of stoppers. Four (4) μl of an 1 M aqueous solution of methanol were added to one of the two test tubes, but nothing was added to the other.

One (1) ml of the bacterial suspension was put in each of the test tubes for 3 minutes-reaction at 45°C. After 3 minutes, 5 μl of a 10 mM aqueous solution of allylthiourea were added. After cooling, the propylene oxide concentration of the solution was measured by gas chromatography. Detection of that concentration was carried out at a carrier gas flow rate of 70 ml $N_2$/min. with a flame ionization detector, while maintaining at 150°C a glass column (of 3 mm in diameter and 2.1 m in length) packed with Gaskuropack 54. The retention time of the product was 3.00 minutes in coincidence with that of a standard sample of propylene oxide. The activity of methane monooxygenase was expressed in terms of the initial reaction rate by the experimental procedure described above. The rate of production of propylene oxide was 320 nmol/ml/min./$O.D._{540}$ = 2 in the presence of methanol added as the electron donor source, whereas that rate of production was 50 nmol/ml/min./O.D.$_{540}$ = 2 in the absence of any electron donor source.

Twenty (20) ml of the aforesaid bacterial suspension were put in a test tube of 60 ml in volume, and 20 $\ell$ of an 1 M aqueous solution of methanol were added, and incubated at 45°C on a reciprocal shaker (200 rpm). Until five hours elapsed, 20 $\mu$l of the methanol solution were added every one hour. At the time five hours elapsed, it was ascertained that the methanol concentration in the test tube was reduced to 0 mM, and a part of the suspension was removed to measure the activity of methane monooxygenase in the aforesaid manner. As a result, it was found that the activity was 240 nmol/ml/min./O.D.$_{540}$ = 2 in the presence of methanol added as the electron donor source, whereas it was 30 nmol/ml/min./O.D.$_{540}$ = 2 in the absence of any electron donor source.

After the measurement of activity, methanol was added in such a manner that its concentration was increased to 1 mM. Afterwards, any methanol was not added at all, until 18 hours elapsed.

At the time 18 hours elapsed, it was ascertained that the methanol concentration of water was reduced to 0 mM, and a part of the bacterial suspension was taken out to measure the activity of methane monooxygenase in the same manner as mentioned above. In consequence, it was found that the activity was 30 nmol/ml/min./O.D.$_{540}$ = 2 in the presence of methanol added as the electron donor source, whereas it was 0 nmol/ml/min./O.D.$_{540}$ = 2 in the absence of any electron donor source.

After the activity was measured in this manner, methanol was added to increase its concentration to 1 mM. Afterwards, methanol was added every 1 hour to control its concentration to 1 mM, until 20 hours elapsed. At the time 20 hours elapsed, it was ascertained that the methanol concentration in the test tube was reduced to 0.00 mM, and a part of the bacterial suspension was taken out to measure the activity of methane monooxygenase in the same manner as mentioned above. In consequence, the activity was found to be 240 nmol/ml/min./O.D.$_{540}$ = 2, when methanol was added as the electron donor source.

Thus, it was clearly possible to recover the activity of the biomasses showing considerably reduced activity by adding methanol thereto.

Example 3

Example 2 was repeated, except that the buffer solution having the following composition was used to wash and suspend the biomasses.

| Tris(hydroxymethyl)aminomethane | 2.4 g |
| 3-(N-morpholino)propane sulfonic acid | 10.5 g |
| Distilled water | 1 $\ell$ |

After the lapse of 18 hours, the activity of methane monooxygenase was 30 nmol/ml/min./O.D.$_{540}$ = 2, when methanol was added as the electron donor source. In the absence of any electron donor source, however, that the activity was decreased to 0 nmol/ml /min./O.D.$_{540}$ = 2.

Methanol was added every 1 hour to control its concentration to 1 mM, until 20 hours elapsed. At the time 20 hours elapsed, the activity of methane monooxygenase was measured. The activity was 240 nmol/ml/min./O.D.$_{540}$ = 2, when methanol was added as the electron donor source. In the absence of any electron donor source, however, that activity was decreased to 20 nmol/ml/min./O.D.$_{540}$ = 2.

Example 4

Example 2 was repeated, except that formaldehyde was added in place of methanol in such a manner that its concentration was again adjusted to 1 mM.

After the lapse of 18 hours, the activity of methane oxidase was reduced to 0 nmol/ml/min./O.D.$_{540}$ = 2, when methanol was added as the electron donor source. Until 2 hours elapsed thereafter, formaldehyde was added every one hour to control its concentration to 1 mM. At the time 20 hours elapsed, the activity of methane monooxygenase was measured. That activity was 200 nmol/ml/min./O.D.$_{540}$ = 2, when methanol was added as the electron donor source.

Thus, it was also possible to recover the activity of methane monooxygenase by the addition of formaldehyde, as was the case with the addition of methanol.

Comparative Example 1

Example 2 was repeated, except that sodium formate was added in such a manner that its concentration was here adjusted to 10 mM, instead of adding methanol at a concentration of 1 mM.

After the lapse of 18 hours, the activity of methane monooxygenase was 40 nmol/ml/min./O.D.$_{540}$ = 2, when methanol was added as the electron donor source. Until 20 hours elapsed, sodium formate was added every one hour to control its concentration to 10 mM. After the lapse of 21 hours, however, the activity of methane monooxygenase was reduced to 30 nmol/ml/min./O.D.$_{540}$ = 2 even in the presence of methanol added as the electron donor source, and could not be recovered. The activity of methane monooxygenase could not again be recovered by adding ethanol in place of sodium formate in such a manner that its concentration was controlled to 1 mM.

Comparative Example 2

Example 2 was repeated, except that Methylococcus capsulatus NCIB 11132 strain was used in place of Methylococcus sp. SIM-222 strain (FERM BP-1470), and 20 mM of a tris-HCl buffer solution (pH 7.0) containing 5 mM of MgCl$_2$ were used to wash and suspend the biomasses.

The initial activity of methane monooxygenase was as high as 314 nmol/ml/min./O.D.$_{540}$ = 2, when methanol was added as the electron donor source.

After the lapse of 18 hours, the activity of methane monooxygenase was reduced to 141 nmol/ml/min./O.D.$_{540}$ = 2, when methanol was added as the electron donor source.

Until 20 hours elapsed thereafter, methanol was added every one hour to control its concentration to 1 mM. After the lapse of 2 hours, the activity of methane monooxygenase was measured. As a result, that the activity was reduced to 140 nmol/ml/min./O.D.$_{540}$ = 2.

With Methylococcus capsulatus NCIB 11132 strain, it was impossible to recover the reduced activity of methane monooxygenase by the addition of methanol.

Example 5

According to the procedures described in the item (2) of Example 1, a culture medium was prepared and sterilized under pressure, and its gaseous phase was replaced by a mixed gas. After than, propylene oxide was added to the medium in such a manner that its concentration in the liquid phase at 45°C was adjusted to 0.1 ml/ℓ. The obtained medium was inoculated with 2.5 ml of the culture solution obtained in the item (2) of Example 1 for 24 hour-shaked incubation at 45°C. At the same time, a propylene oxide-free medium was provided, and was inoculated with 2.5 ml of the same culture solution as used previously for 24 hour-shaked incubation at 45°C.

After the lapse of 24 hours, the turbidity of the culture solution was measured in the same manner as described in the item (2) of Example 1. As a result, it was found that there was no substantial difference in turbidity between both the culture solutions, as proven by the fact that the culture solution free from propylene oxide showed an absorbance of 2.3, whereas the culture solution containing propylene oxide indicated an absorbance of 2.1.

In the same manner as described in Example 2, the activity of methane monooxygenase of each of the cultured biomasses was measured. However, 20 mM of a tris-HCl buffer solution containing 5 mM of MgCl$_2$ were used to wash and suspend each biomass, and 4 ℓ of an 1 M aqueous solution of formaldehyde were used in place of an 1 M aqueous solution of methanol as the electron donor source to be added for the measurement of activity. The activity of the biomass cultured in the absence of propylene oxide was 270 nmol/ml/min./O.D.$_{540}$ = 2, whereas that of the biomass cultured in the presence of propylene oxide was 240 nmol/ml/min./O.D.$_{540}$ = 2. It turned out that the propylene oxide concentration of 0.1 ml/ℓ had no substantial influence upon the growth of Methylococcus sp. SIM-222 strain and the activity of methane monooxygenase.

Comparative Example 3

Example 5 was repeated, except that the strain used was Methylococcus capsulatus NCIB 11132 strain, the culture medium applied was a NMS-medium (J. Gen. Microbiol. (1970), 61, 205-218, R. Whittenbury, K.C. Phillips and J.F. Wilkinson), and the gaseous phase involved consisted of 50% by volume of methane and 50% by volume of air. In cosequence, Methylococcus capsulatus NCIB 11132 was not grown.

10

Example 6

In the same manner as described in the item (2) of Example 1, Methylococcus sp. SIM-222 strain (FERM BP-1470) were cultured. However, methanol was added as the carbon source in such a manner that its concentration was regulated to 2%. After 24 hour-incubation, the culture solution showed an absorbance of 2.3 at 540 nm. The activity of methane monooxygenase was 200 nmol/ml/min./O.D.$_{540}$ = 2.

It was found that, without using the method involving supply of methanol in the vaporized form (Japanese Patent Laid-Open Publication No. 57-65187), Methylococcus sp. SIM-222 strain was vigorously grown and could stably retain the activity of methane monooxigenase in the presence of methanol added at a high concentration.

Example 7

Methylococcus sp. SIM-222 strain (FERM BP-1470) was cultured in the same manner as described in the item (2) of Example 1, except that the copper concentration applied was 10 mg/$\ell$, as calculated as copper sulfate. After the lapse of 24 hours, the culture solution showed an absorbance of 2.0 at 540 nm, and the activity of methane monooxygenase was 220 nmol/ml/min./O.D.$_{540}$ = 2. It was ascertained that Methylococcus sp. SIM-222 strain was vigorously grown and maintained the activity of methane monooxygenase even at such a high copper concentration. Not until now is any report made on the methane-utilizing bacteria capable of growing at such a high copper concentration. Even though other methane assimilative bacteria contaminate with the strain of this invention, they can easily be eliminated.

Example 8

The culture solution obtained according to the procedures described in the item (2) of Example 1 was centrifuged at 5,000 x g for 10 min. to collect biomasses, which were then centrifugally washed twice with 20 mM of a tris-HCl buffer solution containing 5 mM of MgCl$_2$. Suspended in that buffer solution were the biomasses in such a manner that its absorbance was adjusted to 2 at 540 nm. At the respective concentrations of 1 mM, octane and methanol acting as the electron donor source were added to 1 ml of the obtained bacterial suspension, which was then subjected to shaking culture (200 rpm) at 45°C for 30 minutes. As a result, 0.3 mg/$\ell$ of octanol were produced.

Example 9

Example 8 was repeated, except that chlorobenzene was added in place of octane in such a manner that its concentration was controlled to 1 mM. In consequence, 2.56 mg/$\ell$ of p-chlorophenol were obtained.

Comparative Example 4

Incubation was carried out in the same manner as described in the item (2) of Example 1, except that Methylococcus capsulatus NCIB 11132 strain was used in place of Methylococcus sp. SIM-222 strain, a NMS-medium was employed as the culture medium, and methanol was added as the carbon source in such a manner that its concentration was regulated to 2%. After 24 hour-incubation, the culture solution showed an absorbance of 1.2 at 540 nm, and the activity of methane monooxygenase was not found.

Comparative Example 5

Incubation was carried out in the same manner as described in the item (2) of Example 1, except that Methylococcus capsulatus NCIB 11132 starin was employed in place of Methylococcus sp. SIM-222 strain, and the copper concentration applied was controlled to 10 mg/$\ell$, as calculated as copper sulfate. Any growth of biomasses was not observed in 24 hour-incubation.

Example 10

Methylococcus sp. SIM-222 strain (FERM BP-1470) was cultured in the same manner as described in the item (2) of Example 1, except that the culture temperature applied was 52°C, not 45°C. After 24 hour-incubation, the culture solution showed an absorbance of 2.4 at 540 nm, from which it was ascertained that the strain grew vigorously even under high-temperature culture conditions.

Comparative Example 6

Incubation was carried out in the same manner as described in the item (3) of Example 1, except that the strain used was Methylcoccus capsulatus NCIB 11132 strain, and the incubation temperature applied was 52°C. After 24 hours, any growth of biomasses did not occur at all.

Example 11

Methylococcus sp. SIM-222 strain (FERM BP-1470) was cultured in the same manner as described in Example 1-(2), except that penicillin G or ampicillin was added to the culture medium in such a manner that its concentration was regulated to 1.5 units/ml or 2 μg/ml. After the strains had been cultured at 45°C for 24 hours, the culture solution showed absorbance of 2.1 and 2.0 at 540 nm in the presence of penicillin G and ampicillin, respectively. The absorbance was 2.0, when nothing was added. Methylococcus sp. SIM-222 strain retains so strong resistance to penicillin and ampicillin that, even though other methane-utilizing bacteria, e.g., Methylococcus capsulatus NCIB 11083 strain, contaminate to the present strain, they can easily be eliminated.

Comparative Example 7

For incubation at 45°C for 24 hours, Methylococcus capsulatus NCIB 11083 strain was used in place of Methylococcus sp. SIM-222 strain, a NMS-medium (J. Gen. Microbiol. (1970), 61, 205-218, R. Whittenburry, K.C. Phillips and J.F. Wilkinson) was employed, the gaseous phase was composed of 50% by volume of methane and 50% by volume of air, and penicillin G or ampicillin was added to the medium in such a manner that its concentration was respectively controlled to 1.5 units/ml or 2 g/ml. After 24 hours, any growth of the strain was not found at all. Hence, it was clear that Methylococcus capsulatus NCIB 11083 strain was much lower in the resistance to penicillin and ampicillin than Methylococcus sp. SIM-222 strain.

Example 12

Example 8 was repeated, except that 5 ml of propane in place of octane were added in the gaseous phase, and sodium formate was added as the electron donor source in such a manner that its concentration was regulated to 20 mM. As a result, 0.4 mg/ℓ of isopropanol were produced.

Example 13

Example 8 was repeated, except that 5 ml of 1-butene in place of octane were added in the gaseous phase, and sodium formate was added as the electron donor source in such a manner that its concentration was controlled to 20 mM. As a result, 150 nmol/ml/min. of butylene oxide were produced.

Example 14

Example 8 was repeated, except that 5 ml of butane in place of octane were added in the gaseous phase, and sodium formate was added as the electron donor source in such a manner that its concentration was regulated to 20 mM. As a result, 0.05 mg/ℓ of isobutanol were produced.

**Claims**

1. Methylococcus sp. SIM-222 (FERM BP-1470), which belongs to a genus Methylococcus, and is capable of being grown using peptone or casamino acid as the nitrogen source in the presence of methane.

2. A method for producing oxygenous compound, which comprises contacting Methylococcus sp. SIM-222 (FERM BP-1470) with an oxidizable organic compound containing at least two carbon atoms under aerobic conditions.

3. A method as claimed in Claim 2, wherein said oxidizable organic compound containing at least two carbon atoms is a compound selected from the group consisting of alkanes, alkenes, ethers, alicyclic compounds, aromatic compounds and heterocyclic compounds.

4. A method as claimed in Claim 3, wherein said oxidizable organic compound containing at least two carbon atoms is a compound selected from the group consisting of alkanes such as propane, butane, pentane and octane; alkenes such as propene, butene, pentene and octene; ethers such as dimethyl ether, diethyl ether and methyl ethyl ether; alicyclic compounds such as cyclopentane, cyclohexane, cyclooctane, cyclodecane, cyclopentene, cyclohexene, cyclooctene and cyclodecene; aromatic compounds such as benzene, toluene, xylene, biphenyl, indene, naphthalene, tetralin and anthracene; and heterocyclic compounds such as thiophene, pyridine, thiazole, pyrimidine, triazine and quinoline.

5. A method as claimed in Claim 2, wherein the contact is carried out at a temperature of from 25 to 55°C.

## Patentansprüche

1. Methylococcus sp. SIM-222 (FERM BP-1470), der zur Gattung Methylococcus gehört und in der Lage ist bei Verwendung von Pepton oder Casaminosäure als Stickstoffquelle in Gegenwart von Methan zu wachsen.

2. Verfahren zur Herstellung einer Oxydationsverbindung, dadurch gekennzeichnet, daß Methylococcus sp. SIM-222 (FERM BP-1470) mit einer oxydierbaren organischen Verbindung, die wenigstens 2 Kohlenstoffatome enthält, unter aeroben Bedingungen kontaktiert wird.

3. Verfahren nach Anspruch 2, worin die oxydierbare organische Verbindung, die wenigstens 2 Kohlenstoffatome enthält, eine Verbindung ist, die aus der Gruppe ausgewählt wird, die aus Alkanen, Alkenen, Ethern, alicyclischen Verbindungen, aromatischen Verbindungen und heterocyclischen Verbindungen besteht.

4. Verfahren nach Anspruch 3, worin die oxydierbare organische Verbindung, die wenigstens 2 Kohlenstoffatome enthält, eine Verbindung ist, die ausgewählt ist aus der Gruppe, bestehend aus Alkanen wie Propan, Butan, Pentan und Octan; Alkenen wie Propen, Buten, Penten und Octen; Ethern wie Dimethylether, Diethylether und Methylethylether; alicyclischen Verbindungen wie Cyclopentan, Cyclohexan, Cyclooctan, Cyclodecan, Cyclopenten, Cyclohexen, Cyclooncten und Cyclodecen; aromatischen Verbindungen wie Benzen, Toluen, Xylen, Biphenyl, Inden, Naphthalen, Tetralin und Anthracen; und heterocyclischen Verbindungen wie Thiophen, Pyridin, Thiazol, Pyrimidin, Triazin und Chinolin.

5. Verfahren nach Anspruch 2, worin der Kontakt bei einer Temperatur von 25 bis 55 °C durchgeführt wird.

## Revendications

1. Methylococcus sp. SIM-222 (FERM BP-1470), appartenant au genre Methylococcus, et que l'on peut cultiver en utilisant de la peptone ou du casaminoacide comme source d'azote en présence de méthane.

2. Procédé de production de composé oxygéné qui comprend la mise en contact de Methylococcus sp. SIM-222 (FERM BP-1470) avec un composé organique oxydable contenant au moins deux atomes de carbone en conditions aérobies.

3. Procédé selon la revendication 2, dans lequel ledit composé organique oxydable contenant au moins deux atomes de carbone est un composé choisi dans le groupe comprenant les alcanes, les alcènes, les éthers, les composés alicycliques, les composés aromatiques et les composés hétérocycliques.

4. Procédé selon la revendication 3, dans lequel ledit composé organique oxydable contenant au moins deux atomes de carbone est un composé choisi dans le groupe comprenant des alcanes comme le propane, le butane, le pentane et l'octane; des alcènes comme le propène, le butène, le pentène et l'octène; des éthers comme le diméthyléther, le diéthyléther et le méthyléthyléther; des composés alicycliques comme le cyclopentane, le cyclohexane, le cyclooctane, le cyclodécane, le cyclopentène, le cyclohexène, le cyclooctène et le cyclodécène; des composés aromatiques comme le benzène, le toluène, le xylène, le biphényle, l'indène, le naphtalène, la trétraline et l'anthracène; et des composés

EP 0 263 403 B1

hétérocycliques comme le thiophène, la pyridine, le thiazole, la pyrimidine, la triazine et la quinoline.

**5.** Procédé selon la revendication 2, dans lequel le contact est effectué à une température comprise entre 25 et 55°C.

14